# EUROPÄISCHE PATENTANMELDUNG

(11) **EP 0 550 385 A1**
(43) Veröffentlichungstag der Anmeldung: **07.07.1993**
(21) Anmeldenummer: 92810990.9
(22) Anmeldetag: 11.12.1992
(51) Int. Cl.: A61K 31/66, A61K 31/675, A61K 31/335

(54) **Peroral zu verabreichende pharmazeutische Zusammensetzungen, die Methandiphosphonsäure-Derivate und 18-Krone-6 Ether enthalten**

(30) Priorität: 19.12.1991 CH 3779/91; 27.01.1992 CH 215/92
(71) Anmelder: CIBA-GEIGY AG, 4002 Basel (CH)
(72) Erfinder: Flesch, Gérard, Dr., F-68100 Mulhouse (FR); Lehn, Jean-Marie, Prof. Dr., F-67000 Strasbourg (FR)

(57) **Zusammenfassung**

Die Erfindung betrifft peroral zu verabreichende pharmazeutische Zusammensetzungen enthaltend (a) pharmazeutisch verwendbare Methandiphosphonsäure-Derivate, z.B. solche von der Formel I, 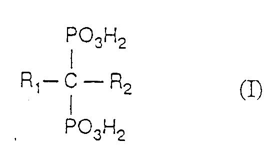
worin R₁ und R₂ wie in der Beschreibung angegeben definiert sind, oder pharmazeutisch annehmbare Salze davon, (b) makrozyklische Polyether der Formel II 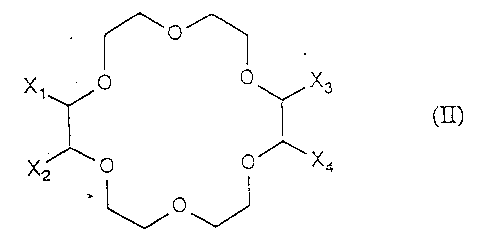
worin X&sub1;-X&sub4; unabhänig voneinander Carboxy, Carbamoyl oder N-mono- oder N,N-di-substituiertes Carbamoyl bedeuten, und (c) gegebenenfalls pharmazeutisch annehmbare Hilfsstoffe. Sie werden in an sich bekannter Weise hergestellt.

## Beschreibung

Methandiphosphonsäure-Derivate werden bekanntlich z.B. zur Behandlung von osteolytischen Knochenmetastasen und Hyperkalzämie eingesetzt, da sie in der Lage sind, das Wachstum und die Zersetzung von Hydroxyapatit zu hemmen. Diese Verbindungen hemmen die Knochenresorption, indem sie sich spontan an das Hydroxyapatit des Knochens binden, so dass z.B. Osteoklasten keine Hydroxyapatitkristalle mehr abspalten können. Verbindungen dieser Substanzklasse werden beispielsweise in der DE-OS-2 405 254 beschrieben.

Es ist bekannt, dass Methandiphosphonsäure-Derivate nach peroraler Verabreichung nur in geringem Masse im Magen-Darm-Trakt resorbiert werden. Um den gewünschten therapeutischen Effekt zu erreichen, müssen sie dementsprechend peroral in höherer Dosierung oder parenteral verabreicht werden.

Wegen der Bedeutung dieser Substanzklasse für die Behandlung beispielsweise von Osteoporose, Morbus Paget, Morbus Bechterew sowie Knochenmetastasenbildungen liegt das Ziel vielfacher Bemühungen darin, eine perorale pharmazeutische Applikationsform bereitzustellen, bei der die Wirkstoffe in grösserem Ausmass resorbiert werden.

Es wurde nun überraschenderweise gefunden, dass Methandiphosphonsäure-Derivate nach peroraler Verabreichung wesentlich besser resorbiert werden, wenn sie zusammen mit bestimmten makrozyklischen Polyethern verabreicht werden.

Gegenstand der vorliegenden Erfindung sind daher peroral zu verabreichende pharmazeutische Zusammensetzungen enthaltend
(1) mindestens ein pharmazeutisch verwendbares Methandiphosphonsäure-Derivat,
(2) mindestens einen makrozyklischen Polyether der Formel II, 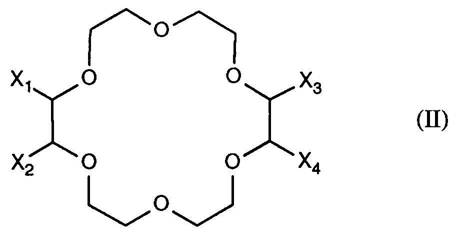 worin X₁-X₄ unabhängig voneinander Carboxy, Carbamoyl oder N-mono- oder N,N-di-substituiertes Carbamoyl bedeuten, oder ein pharmazeutisch annehmbares Salz davon, und
(3) gegebenfalls pharmazeutisch annehmbare Hilfsstoffe.

Ein pharmazeutisch verwendbares Methandiphosphonsäure-Derivat besitzt z.B. die Struktur der Formel I, 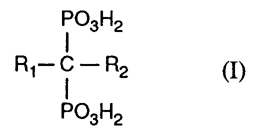
worin R₁ Wasserstoff, Hydroxy, Amino oder Halogen bedeutet und R₂ für Halogen oder einen über C, N, S oder O gebundenen Rest steht, und umfasst auch entsprechende pharmazeutisch annehmbare Salze.

Bevorzugt sind pharmazeutische Zusammensetzungen, die als Methandiphosphonsäure-Derivat mindestens eine Verbindung der Formel I, worin
(a) R₁ und R₂ unabhängig voneinander Halogen bedeuten, oder
(b) R₁ Wasserstoff ist und R₂ eine Gruppe Ar-S-, Het₁-NH-, Cyc-NH-, Ar-S-A-N(R')- oder Het₃-S-A-N(R')- bedeutet, worin Ar für unsubstituiertes oder substituiertes Phenyl steht, Het₁ und Het₃ jeweils unsubstituiertes oder substituiertes, monocyclisches 5- oder 6-gliedriges Monoaza-, Diaza- oder Thiaza-aryl bedeuten, welches über ein Ringkohlenstoffatom gebunden ist, Cyc für Cycloalkyl steht, A Alkylen bedeutet und R' für Wasserstoff oder Niederalkyl steht, oder
(c) R₁ Wasserstoff oder Hydroxy bedeutet und R₂ für -A-R₃ steht, worin A Alkylen bedeutet und
   R₃ entweder für Het₂ steht, das wie Het₁ definiert ist, aber über ein Ringkohlenstoff- oder ein Ringstickstoffatom gebunden sein kann, oder
   R₃ Wasserstoff oder unsubstituiertes oder durch Alkyl, Cycloalkyl, Ar-Alkyl, Ar-O-Alkyl, Ar-S-Alkyl oder Het₁-Alkyl unabhängig voneinander mono- oder disubstituiertes Amino bedeutet, wobei Ar und Het₁ wie oben definiert sind, oder
   R₃ für durch unsubstituiertes oder durch Ar substituiertes Alkylenamino steht, worin zwei Alkylenkohlenstoffatome zusätzlich über Alkylen miteinander verknüpft sein können und Ar wie oben definiert ist,
   oder ein pharmazeutisch annehmbares Salz davon, enthalten.

Salze von Verbindungen der Formel I sind insbesondere deren Salze mit Basen, wie von Gruppen Ia, Ib, IIa und IIb des Periodensystems der Elemente abgeleitete Metallsalze, z.B. Alkalimetall-, insbesondere Natrium- oder Kaliumsalze, Erdalkalimetall-, insbesondere Calcium- oder Magnesiumsalze, ebenso z.B. Ammoniumsalze mit Ammoniak oder organischen Aminen.

Substituiertes Phenyl ist ein- oder mehrfach, z.B. zwei-, ferner dreifach, substituiert, z.B. durch Niederalkyl, Niederalkoxy, Trifluormethyl und/oder insbesondere Halogen.

Monocyclisches, 5- oder 6-gliedriges Monoaza-, Diaza- oder Thiazaaryl bedeutet beispielsweise Pyrrolyl, Imidazolyl, wie 1-, 2-, 4- oder 5-Imidazolyl, Pyrazolyl, wie 1- oder 3-Pyrazolyl, Thiazolyl, wie 2- oder 4-Thiazolyl, Pyridyl, wie 2-, 3- oder 4-Pyridyl. Entsprechende Reste können z.B. durch Alkyl ein- oder mehrfach, z.B. zwei-, ferner dreifach, substituiert sein. Bevorzugte substituierte Reste sind beispielsweise Alkyl-substituiertes 1-Imidazolyl und 5-Imidazolyl, 5-Niederalkyl-2-thiazolyl, wie 5-Methyl-2-thiazolyl, 5-Ethyl-2-thiazolyl und 5-n-Butyl-2-thiazolyl, sowie Alkyl-substituiertes 2- und 3-Pyridyl.

Unsubstituiertes oder substituiertes, monocyclisches 5- oder 6-gliedriges Monoaza-, Diaza- oder Thiaza-aryl, welches über ein Ringkohlenstoffatom gebunden ist, bedeutet insbesondere einen Rest aus der Gruppe 2-, 4- oder 5-Imidazolyl, 3-Pyrazolyl, Thiazolyl, z.B. 2- oder 4-Thiazolyl, und Pyridyl, z.B. 2-, 3- oder 4-Pyridyl, der unsubstituiert oder durch Niederalkyl substituiert ist.

Unsubstituiertes oder substituiertes, monocyclisches 5- oder 6-gliedriges Monoaza-, Diaza- oder Thiaza-aryl, welches über ein Ringkohlenstoff- oder ein Ringstickstoffatom gebunden ist, bedeutet insbesondere einen Rest aus der Gruppe Pyrrolyl, Imidazolyl, Pyrazolyl, Thiazolyl oder Pyridyl, der unsubstituiert oder durch Niederalkyl substituiert ist.

Alkyl ist insbesondere Niederalkyl und Alkylen insbesondere Niederalkylen, während Ar-Alkyl beispielsweise gegebenenfalls im Phenylteil wie vorstehend angegeben substituiertes Phenylniederalkyl bedeutet.

Cycloalkyl ist insbesondere C₃-C₇-Cycloalkyl, wie Cyclopropyl, -butyl, -pentyl, -hexyl oder -heptyl.

Durch Alkyl, Cycloalkyl, Ar-Alkyl, Ar-O-Alkyl, Ar-S-Alkyl oder Het₁ -Alkyl mono- oder disubstituiertes Amino bedeutet insbesondere Niederalkyl-, C₃-C₇-Cycloalkyl-, Phenylniederalkyl-, Diniederalkyl-, Niederalkyl-phenylniederalkyl-, Diphenylniederalkylamino, Phenoxyniederalkylamino, Niederalkyl-phenoxyniederalkyl-amino, Phenoxyniederalkyl-phenylniederalkyl-amino, Diphenoxyniederalkyl-amino, Phenylthioniederalkyl-, Niederalkyl-phenylthioniederalkyl-, Phenylthioniederalkyl-phenylniederalkyl-, Diphenylthioniederalkyl-amino, Niederalkyl-pyridylniederalkyl-amino, Phenylniederalkyl-pyridylniederalkylamino, Phenoxyniederalkyl-pyridylniederalkylamino, Phenylthioniederalkyl-pyridylniederalkyl-amino oder Dipyridylniederalkylamino, wobei der Phenyl- bzw. Pyridylteil gegebenenfalls wie vorstehend angegeben substituiert sein kann.

Gegebenenfalls durch Ar substituiertes Alkylenamino bedeutet insbesondere Niederalkylenamino, z.B. 1,4-Butylenamino (= Pyrrolidin-1-yl) oder 1,5-Pentylenamino (= Piperidin-1-yl), oder durch einen unsubstituierten oder wie vorstehend angegeben substituierten Phenylrest substituiertes Niederalkylenamino, wie 2-(4-Chlorphenyl)- 1,4-butylen-amino oder 3-Phenyl- 1,5-pentylen-amino.

Alkylenamino, worin zwei Alkylenkohlenstoffatome zusätzlich über Alkylen miteinander verknüpft sind, bedeutet insbesondere Niederalkylenamino, worin zwei, insbesondere nicht benachbarte, Niederalkylenkohlenstoffatome über Niederalkylen, in erster Linie Methylen, miteinander verknüpft sind. Bevorzugt sind z.B. entsprechende 3-Azabicyclo-C₆-C₁₀-alk-3-yl-Reste.

Die vor- und nachstehend verwendeten Allgemeindefinitionen haben, sofern nicht abweichend definiert, in erster Linie die folgenden Bedeutungen:

Halogen ist insbesondere Halogen mit Atomnummer bis und mit 35, wie Fluor oder Brom, ferner Jod, in erster Linie Chlor.

Niederalkyl ist z.B. Methyl, Ethyl, n-Propyl, Isopropyl, n-Butyl, Isobutyl, sek- und tert-Butyl und umfasst ferner entsprechende Pentyl-, Hexyl- und Heptylreste. Bevorzugt ist C₁-C₄-Alkyl.

Niederalkoxy ist z.B. Methoxy, Ethoxy, n-Propyloxy, Isopropyloxy und n-Butyloxy und umfasst ferner entsprechende Pentyloxy-, Hexyloxy- und Heptyloxyreste. Bevorzugt ist C₁-C₄-Alkoxy.

Niederalkylen ist geradkettig oder verzweigt und bedeutet z.B. C₁-C₇-Alkylen, wie Methylen, Ethylen, Propylen, Butylen, Pentylen, ferner Hexylen und Heptylen, sowie 2-Methyl- 1,3-propylen, 2,4- oder 1,5-Dimethyl- 1,5-pentylen. Niederalkylen als Substituent von disubstituiertem Amino R₃ weist mindestens zwei C-Atome, vorzugsweise 4-6 C-Atome, auf. Niederalkylen, welches zwei Niederalkylenkohlenstoffatome von durch Niederalkylen disubstituiertem Amino miteinander verknüpft, weist insbesondere bis und mit 5 C-Atome auf und bedeutet bevorzugt Methylen.

C₁-C₃-Alkyl ist Methyl, Ethyl, n-Propyl und Isopropyl.

Carbamoyl steht für die Gruppe -CONH₂.

N-mono-substituiertes Carbamoyl ist z.B. N-Niederalkylcarbamoyl, N-(Aminoniederalkyl)-carbamoyl; N-Arylcarbamoyl, worin Aryl für Phenyl oder Naphthyl steht, welche jeweils unsubstituiert oder durch Carboxy und/oder -SO₃H substituiert sind; N-[(Niederalkoxycarbonyl oder Carboxy)-niederalkyl]-carbamoyl oder N-[(3-Carbamoyl-1-pyridinium)-niederalkyl]-carbamoyl.

N,N-di-substituiertes Carbamoyl ist z.B. N,N-Diniederalkylcarbamoyl.

Weiterhin umfassen N-mono- und N,N-di-substituiertes Carbamoyl z.B. solche Reste, bei denen das Amidstickstoffatom von einer alpha-Aminosäure gebildet wird und daher entsprechend monosubstituiert (bzw. z.B. im Falle von Prolin disubstituiert) ist. Hervorzuheben sind dabei die von Glycin, Prolin, Phenylalanin, Tryptophan, Glutamminsäure und Arginin abgeleiteten Carbamoylreste. In diesen können die Carboxygruppen der jeweiligen Aminosäure frei, in Salzform oder auch derivatisiert, z.B. als Niederalkylester oder Carboxamide, vorliegen.

Mit "nieder" bezeichnete Reste oder Verbindungen weisen z.B. bis und mit 7 C-Atome, insbesondere bis und mit 4 C-Atome, auf.

Bevorzugt als makrozyklische Polyether der Formel II sind diejenigen, bei denen die Reste X₁-X₄ identisch sind.

Insbesondere bevorzugt als makrozyklische Polyether der Formel II sind diejenigen der Formel IIa, 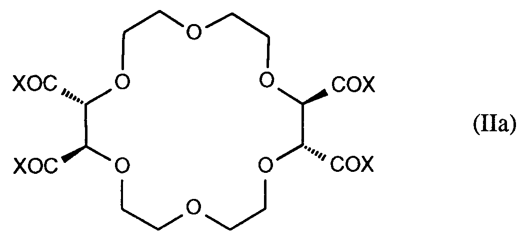
worin die Reste X identisch sind und für Hydroxy stehen, sowie pharmazeutisch verwendbare Salze davon. Es handelt sich hierbei um die (+)-1,4,7,10,13,16-Hexaoxacyclooctadecan-2,3, 11,2-tetracarbonsäure [abgekürzt: (+)- 18-Krone-6-tetracarbonsäure] und pharmazeutisch verwendbare Salze davon. In erster Linie ist die freie (+)- 1,4,7,10,13,16-Hexaoxa-cyclooctadecan-2,3,11,12-tetracarbonsäure bevorzugt.

In den pharmazeutischen Zusammensetzungen gemäss der Erfindung liegen die Komponenten (1) [ = pharmazeutisch verwendbare(s) Methandiphosphonsäure-Derivat(e)] und (2) [ = makrozyklische(r) Polyether] vorzugsweise in einem Verhältnis von 1:1 bis 1:16, insbesondere 1:1 bis 1:8, und vor allem 1:2 bis 1:6 vor.

Die pharmazeutisch verwendbaren Methandiphosphonsäure-Derivate sind bekannt oder können in an sich bekannter Weise hergestellt werden.

So sind beispielsweise Verbindungen der Formel I, worin R₁ Wasserstoff ist und R₂ Ar-S-bedeutet, durch Umsetzung von Tetraniederalkylmethandiphosphonat in Gegenwart einer starken Metallbase, wie NaH, mit einem Disulfid der Formel Ar-S-S-Ar und sich anschliessender saurer Hydrolyse des Tetraniederalkylesters zugänglich (s. z.B. EP-A-100 718).

Entsprechende Verbindungen der Formel I, worin R₁ Wasserstoff ist und R₂ Het₁ -NH-bedeutet, kann man z.B. herstellen, indem man ein Gemisch aus H₃PO₃ und PHal₃, worin Hal Halogen, insbesondere Chlor, bedeutet, mit einem Formylamin der Formel Het₁-NH-CHO umsetzt, oder indem man ein Amin Het₁-NH₂ mit einem Orthoameisensäureniederalkylester sowie einem Diniederalkylphosphit erhitzt und das Reaktionsprodukt, z.B. in Gegenwart einer Säure, hydrolysiert (s. z.B. EP-A-274 346).

Zur Herstellung von Verbindungen der Formel I, worin R₁ Wasserstoff ist und R₂ für -A-R₃ steht und A Alkylen bedeutet, kann man beispielsweise von Verbindungen der Formel R₃-A-Hal ausgehen und diese mit einem Tetraniederalkylmethandiphosphonat in Gegenwart einer starken Base, z.B. NaH, umsetzen und den so erhältlichen Tetraniederalkylester von entsprechenden Verbindungen der Formel I z.B. in Gegenwart einer Säure, wie Chlorwasserstoffsäure, hydrolysieren (s. z.B. EP-A-275 821).

Verbindungen der Formel I, worin R₁ Hydroxy bedeutet und R₂ für -A-R₃ steht, kann man beispielsweise herstellen, indem man eine Carbonsäure der Formel R₂-COOH mit einem Phosphorylierungsmittel, wie einem Gemisch aus H₃PO₃ und PHal₃, umsetzt und unter hydrolytischen Bedingungen aufarbeitet (s. z.B. EP-A-170 228 und EP-A-252 505).

Die Herstellung von Verbindungen der Formel Ia (s. u.) sowie pharmazeutisch annehmbaren Salzen davon ist z.B. in der DE-OS-2 405 254 beschrieben.

Die Herstellung der makrozyklischen Polyether der Formel II ist z.B. in J.-P. Behr et al., Helv. Chim. Acta 65, 1853- 1867 (1982) oder J.-P. Behr et al., Helv. Chim. Acta 63, 2096-2111 (1980) beschrieben. Beispielsweise kann man eine Verbindung der Formel II mit X₁= X₂= X₃= X₄= CON(CH₃)₂ so herstellen, indem man N,N,N',N'-Tetramethyltartramid zunächst mit Thallium(I)ethoxid und dann mit 1,5-Diiod-3-oxapentan umsetzt. Die so erhaltene Verbindung kann z.B. durch saure Hydrolyse, z.B. mit Salzäure, in eine andere Verbindung der Formel II, worin X₁, X₂, X₃ und X₄ Carboxy bedeuten, umgewandelt werden.

Die pharmazeutisch verwendbaren Methandiphosphonsäure-Derivate weisen wertvolle pharmakologische Eigenschaften auf. Insbesondere besitzen sie eine ausgeprägte regulierende Wirkung auf den Calcium-Stoffwechsel von Warmblütern. Ebenso bewirken sie an der Ratte eine ausgeprägte Hemmung der Knochenresorption, die sich sowohl in der Versuchsanordnung gemäss Acta Endocrinol. 78, 613-24 (1975) als auch im TPTX (Thyroparathyroidectomised) - Rattenmodell anhand der durch Vitamin D₃ ausgelösten experimentellen Hyperkalzämie zeigen list. Ebenso wird die durch Walker-256-Tumore induzierte Tumorhyperkalzämie nach peroraler Verabreichung gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) eine deutliche Hemmung auf das Fortschreiten chronisch-arthritischer Prozesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, Neuritis, Bursitis, Periodontitis und Tendinitis, Fibrodysplasie, Osteoarthrose oder Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, Morbus Paget und die Osteodystrophia fibrosa, ebenso durch Tumore ausgelöste osteolytische Prozesse sowie Hypercalcämie. Einzelne Vertreter der vorstehend definierten Substanzklasse werden bereits therapeutisch eingesetzt.

Die gastrointestinale Absorption des Wirkstoffes kann z.B. über die Urinausscheidung des Wirkstoffes im Zeitraum 0-96 h nach einer einmaligen peroralen Gabe der erfindungsgemässen pharmazeutischen Zusammensetzungen, z.B. beim Hund, ermittelt werden. Dabei ergibt sich, dass die resorbierte Wirkstoffdosis bei Verabreichung der erfindungsgemässen pharmazeutischen Zusammensetzungen um einen Faktor von etwa 10 oder mehr gesteigert wird gegenüber der Verabreichung des Wirkstoffs etwa in einer "enteric coated" Tablette oder in Form von "enteric coated" Pellets.

Die Erfindung betrifft insbesondere pharmazeutische Zusammensetzungen enthaltend mindestens ein pharmazeutisch verwendbares Methandiphosphonsäure-Derivat der Formel I, worin
(a) R₁ und R₂ unabhängig voneinander Halogen bedeuten, oder
(b) R₁ Wasserstoff ist und R₂ eine Gruppe Ar-S-, Het₁-NH-, Cyc-NH-, Ar-S-A-NH- oder Het₃-S-A-NH- bedeutet, worin Ar für unsubstituiertes oder durch Niederalkyl, Niederalkoxy, Trifluormethyl und/oder Halogen substituiertes Phenyl steht, Het₁ unsubstituiertes oder durch Niederalkyl substituiertes Thiazolyl bedeutet, Cyc für C₃-C₇-Cycloalkyl steht, A Niederalkylen bedeutet und Het₃ für Thiazolyl oder Pyridyl steht, welches jeweils unsubstituiert oder durch Niederalkyl substituiert ist, oder
(c) R₁ Wasserstoff oder Hydroxy bedeutet und R₂ für -A-R₃ steht, worin A Niederalkylen bedeutet und
   R₃ entweder unsubstituiertes oder durch Niederalkyl substituiertes Imidazolyl, welches über ein Ringkohlenstoff- oder ein Ringstickstoffatom gebunden ist, oder Pyridyl bedeutet, oder
   R₃ Wasserstoff oder unsubstituiertes oder durch Niederalkyl, C₃-C₇-Cycloalkyl, Ar-Niederalkyl, Ar-O-Niederalkyl, Ar-S-Niederalkyl oder Pyridylniederalkyl unabhängig voneinander mono- oder di-substituiertes Amino bedeutet, wobei Ar wie oben definiert ist, oder
   R₃ für durch unsubstituiertes oder durch Ar substituiertes C₄-C₆-Alkylenamino steht, worin zwei nicht benachbarte Niederalkylenkohlenstoffatome zusätzlich über C₁-C₃-Alkylen miteinander verknüpft sein können und Ar wie oben definiert ist,
   oder ein pharmazeutisch annehmbares Salz davon.

Die Erfindung betrifft vorteilhaft pharmazeutische Zusammensetzungen enthaltend eine pharmazeutisch verwendbare Methandiphosphonsäure der Formel I, worin
(a) R₁ und R₂ Halogen bedeuten, oder
(b) R₁ Wasserstoff ist und R₂ unsubstituiertes oder durch Halogen substituiertes Phenylthio, unsubstituiertes oder durch Niederalkyl substituiertes Thiazolylamino oder C₅-C₇-Cycloalkylamino bedeutet, oder
(c) R₁ Wasserstoff oder Hydroxy bedeutet und R₂ für -A-R₃ steht, worin A C₁ -C₇-Alkylen bedeutet und R₃ über ein Ringkohlenstoff- oder Ringstickstoffatom gebundenes, unsubstituiertes oder durch Niederalkyl substituiertes Imidazolyl, oder Pyridyl bedeutet, oder
(d) R₁ Hydroxy ist und R₂ für -A-R₃ steht, worin A C₁-C₇-Alkylen bedeutet und R₃ Amino, Di-C₁-C₅-alkylamino, N-C₃-C₇-Cycloalkylamino, N-C₁ -C₄-Alkyl-N-phenyl-C₁-C₅-alkylamino, N-C₁-C₄-Alkyl-N-phenoxy-C₁-C₄-alkyl, N-C₁ -C₄-Alkyl-N-phenyl-thio-C₁-C₄-alkyl, N-C₁-C₄-Alkyl-N-pyridyl-C₁-C₄-alkylamino; C₄-C₆-Alkylenamino, welches unsubstituiert oder durch Phenyl, das seinerseits unsubstituiert oder durch Halogen substituiert ist, substituiert ist; oder 1,5-Di-C₁-C₄-alkyl-3-aza-bicyclo[3.1.1]-hept-3-yl bedeutet, oder
(e) R₁ Hydroxy ist und R₂ für -A-R₃ steht, worin A C₁-C₄-Alkylen bedeutet und R₃ Wasserstoff ist,
   oder ein pharmazeutisch annehmbares Salz davon.

Die Erfindung betrifft sehr bevorzugt pharmazeutische Zusammensetzungen enthaltend eine pharmazeutisch verwendbare Methandiphosphonsäure der Formel I, worin
(a) R₁ und R₂ Chlor bedeuten, oder
(b) R₁ Wasserstoff ist und R₂ unsubstituiertes oder durch Chlor substituiertes Phenylthio, unsubstituiertes oder durch C₁-C₄-Alkyl substituiertes Thiazolylamino oder Cycloheptylamino bedeutet, oder
(c) R₁ Wasserstoff oder Hydroxy bedeutet und R₂ für -A-R₃ steht, worin A Methylen, Ethylen, Propylen oder Pentylen bedeutet und R₃ Imidazol-1-yl, Imidazol-5-yl, 1-Methylimidazol-2-yl, 4-Methyl-imidazol-5-yl, oder 2- oder 3-Pyridyl bedeutet, oder
(d) R₁ Hydroxy ist und R₂ für -A-R₃ steht, worin A Methylen, Ethylen, Propylen oder Pentylen bedeutet und R₃ Amino, Dimethylamino, N-Methyl-N-n-propylamino, N-Methyl-N-n-pentylamino, N-Cycloheptylamino, N-Methyl-N-(2-phenylethyl)-amino, N-Methyl-N-(3-phenylpropyl)-amino oder N-Methyl-N-(5-phenylpentyl)-amino, N-Methyl-N-(3-phenoxypropyl)-amino, N-Methyl-N-(2-phenylthioethyl)-amino, N-Methyl-N-(3-phenylthiopropyl)-amino, N-Methyl-N-[3-(2-pyridyl)-propyl]-amino, Piperidin-1-yl, welches unsubstituiert oder in 4-Stellung durch Phenyl substituiert ist, oder Pyrrolidin-1-yl, welches unsubstituiert oder in 3-Stellung durch 4-Chlorphenyl substituiert ist, oder 1,5-Dimethyl-3-aza-bicyclo[3.1.1]hept-3-yl bedeutet, oder
(e) R₁ Hydroxy ist und R₂ für -A-R₃ steht, worin A Methylen bedeutet und R₃ Wasserstoff ist,
   oder ein pharmazeutisch annehmbares Salz davon.

Die Erfindung betrifft in erster Linie pharmazeutische Zusammensetzungen, welche eine pharmazeutisch wirksame Methandiphosphonsäure ausgewählt aus der folgenden Gruppe von Verbindungen der Formel I enthalten:

4-Amino-1-hydroxy-butan-1,1-diphosphonsäure, 6-Amino-1 -hydroxy-hexan-1,1-diphosphonsäure, 3-(N-Methyl-N-n-pentyl-amino)-1-hydroxy-propan-1,1-diphosphonsäure, 3-[N-(3-Phenylpropyl)-N-methyl-amino]-1-hydroxy-propan-1,1-diphosphonsäure, 3-(N-Methyl-N-5-phenylpentyl-amino)-1-hydroxypropan-1,1-diphosphonsäure, 3- [N-Methyl-N-3-(2-pyridyl)-propyl-amino]-1-hydroxy-propan-1,1-diphosphonsäure, 1 -Hydroxy-3-[N-methyl-N-(3-phenoxypropyl)-amino]-propan-1,1-diphosphonsäure, 1-Hydroxy-3-[N-methyl-N-(2-phenoxyethyl)-amino]-propan-1,1-diphosphonsäure, 4-(4-Phenyl-piperidin-1-yl)-1-hydroxy-butan-1,1-diphosphonsäure, 1-Hydroxy-3-(1-piperidino)-propan-1,1-diphosphonsäure, 1-Hydroxy-3- [3-(4-chlorphenyl)-pyrrolidin-1-yl]-propan-1,1-diphosphonsäure, 1-Hydroxy-2-(imidazol-1-yl)-ethan-1,1-diphosphonsäure, 2-(1 -Methyl-imidazol-2-yl)-ethan-1,1-diphosphonsäure, 1-Hydroxy-2-(4-methyl-imidazol-5-yl)-ethan-1,1-diphosphonsäure, 1-Hydroxy-2-(imidazol-5-yl)-ethan-1,1-diphosphonsäure, 1 -Hydroxy-2-(3-pyridyl)-ethan-1,1-diphosphonsäure, 2-(2-Pyridyl)-ethan-1,1-diphosphonsäure, 1-[(5-n-Butyl-2-thiazolyl)-amino]-methan-1,1-diphosphonsäure, 1 -[(5-Methyl-2-thiazolyl)-amino]-methan-1,1-diphosphonsäure, 1-[(2-Thiazolyl)-amino]-methan- 1,1-diphosphonsäure, 1-Hydroxy-ethan-1,1-diphosphonsäure, 1-(4-Chlor-phenylthio)-methan-1,1-diphosphonsäure, 1,1-Dichlormethan-1,1-diphosphonsäure, 3-(1,5-Dimethyl-3-azabicyclo[3.1.1]hept-3-yl)-1-hydroxy-propan-1,1-diphosphonsäure, 1-[(5-Ethyl-2-thiazolyl)-amino]-methan-1,1-diphosphonsäure, 3-[N-(2-Phenylethyl)-N-methyl-amino]-1-hydroxy-propan-1,1-diphosphonsäure, 3-[N-(2-Phenylthioethyl)-N-methyl-amino]-1-hydroxy-propan-1,1-diphosphonsäure, 3-[N-(3-Phenylthiopropyl)-N-methyl-amino]-1-hydroxy-propan-1,1-diphosphonsäure, 1-Hydroxy-3-(pyrrolidin-1-yl)-propan-1,1-diphosphonsäure, 2-(N-Cycloheptylamino)-ethan- 1,1-diphosphonsäure, 1-(N-Cycloheptylamino)-methan-1,1-diphosphonsäure, 2-(N,N-Dimethylaniino)-1 -hydroxy-ethan-1,1-diphosphonsäure, 3-Amino-1-hydroxy-propan-1,1-diphosphonsäure, 3-(N,N-Dimethylamino)-1-hydroxypropan-1,1-diphosphonsäure, 3-(N-Methyl-N-n-propyl-amino)-1-hydroxypropan-1,1-diphosphonsäure, 1-[N-(4-Phenylthiobutyl)-amino]-methan-1,1-diphosphonsäure oder 1-{N-[4-(2-Pyridyl)thiobutyl]-amino}-methan-1,1-diphosphonsäure, oder jeweils ein pharmazeutisch verwendbares Salz davon, enthalten.

Ein besonderer Gegenstand der Erfindung sind die pharmazeutische Zusammensetzungen, welche als pharmazeutisch wirksame Methandiphosphonsäure eine Verbindung der Formel Ia, 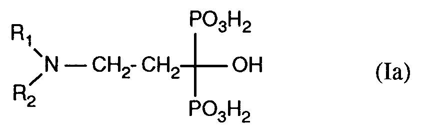
worin R₁ und R₂ unabhängig voneinander Wasserstoff oder C₁-C₃-Alkyl bedeuten, oder ein pharmazeutisch verwendbares Salz davon, enthalten.

Vor allen Dingen betrifft die Erfindung pharmazeutische Zusammensetzungen, welche als Verbindung der Formel I das Dinatriumsalz der 3-Amino-1-hydroxy-propan-1, 1-diphosphonsäure - im folgenden Dinatriumpamidronat (engl. Freiname disodium pamidronate) genannt - und als makrozyklischen Polyether die (+)-1,4,7,10,13,16-Hexaoxacyclooctadecan-2,3,11,12-tetracarbonsäure, oder ein pharmazeutisch anwendbares Salz davon, enthalten.

Gegenstand der Erfindung ist ebenso ein Verfahren zur Herstellung der erfindungsgemässen pharmazeutischen Zusammensetzungen, welches nach an sich bekannter Methodik erfolgen kann und dadurch gekennzeichnet ist, dass man ein pharmazeutisch verwendbares Methandiphosphonsäure-Derivat und einen makrozyklischen Polyether der Formel II mit üblichen pharmazeutisch verwendbaren Hilfs- und Zusatzstoffen verarbeitet.

Ein weiterer Gegenstand der Erfindung ist ein Verfahren zur Steigerung der peroralen Resorption von pharmazeutisch verwendbaren Methandiphosphonsäure-Derivaten, welches dadurch gekennzeichnet ist, dass man ein pharmazeutisch verwendbares Methandiphosphonsäure-Derivat zusammen mit einem makrozyklischen Polyether der Formel II wie oben definiert in eine pharmazeutische Zusammensetzung einarbeitet.

Die Erfindung betrifft vor allen Dingen die in den Beispielen beschriebenen pharmazeutischen Präparate sowie Verfahren zu deren Herstellung.

Die pharmazeutisch verwendbaren Methandiphosphonsäure-Derivate können je nach Wahl der Ausgangsstoffe und Arbeitsweisen in Form eines möglichen Isomeren oder eines Gemisches derselben vorliegen, beispielsweise als optische Isomere, wie Enantiomere oder Diastereomere, oder geometrische Isomere, wie cis-trans-Isomere. Dabei liegen die optischen Isomeren in Form der reinen Antipoden und/oder als Racemate vor.

Die pharmazeutisch verwendbaren Methandiphosphonsäure-Derivate können auch in Form ihrer Hydrate eingesetzt werden oder andere zur Kristallisation verwendete Lösungsmittel einschliessen.

Die erfindungsgemässen peroral zu verabreichenden pharmazeutischen Zusammensetzungen enthalten die pharmazeutisch verwendbaren Methandiphosphonsäure-Derivate beispielsweise in einer pharmakologisch wirksamen Menge. Die tägliche Dosierung des Wirkstoffs hängt vom Alter und individuellen Zustand sowie von der Applikationsweise ab.

Im Normalfall ist bei einem 75 kg schweren Warmblüter von einer ungefähren Tagesdosis von 0,2 bis 200 mg/kg, insbesondere 1 bis 50 mg/kg, auszugehen, die in einer Dosis oder in mehreren Teildosen verabreicht werden kann.

In erster Linie kommen dabei Kapseln, z.B. harte oder weiche Gelatinekapseln, Sachets, Tabletten, beschichtete Tabletten, z.B. Tabletten mit Magensaft-resistenten Ueberzügen ("enteric coated tablets"), oder Dragee-Kerne in Frage, ferner z.B. auch Lösungen.

Einzeldosierte Applikationsformen enthalten vorzugsweise etwa 1 % bis etwa 90 % und nicht-einzeldosierte Applikationsformen vorzugsweise etwa 0,1 % bis etwa 20 % des Gemisches Wirkstoffmakrozyklischer Polyether. Dosiseinheitsformen, wie Kapseln, Tabletten oder Dragees, enthalten z.B. von etwa 1 mg bis etwa 500 mg des Gemisches Wirkstoffmakrozyklischer Polyether.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahen hergestellt. So kann man pharmazeutische Zusammensetzungen zur peroralen Anwendung z.B. erhalten, indem man den Wirkstoff und den makrozyklischen Polyether mit einem oder mehreren festen Trägerstoffen kombiniert und ein erhaltenes Gemisch gegebenenfalls granuliert. Das Gemisch bzw. Granulat kann - falls gewünscht - mit einer oder mehreren Schichten umhüllt werden, wobei man beschichtete Pellets erhält. Das Gemisch bzw. Granulat bzw. die Pellets können, wenn erwünscht, gegebenenfalls durch Zugabe von zusätzlichen Hilfsstoffen, zu Tabletten oder Dragée-Kernen verarbeitet werden.

Geeignete Trägerstoffe sind insbesondere Füllstoffe, wie Zucker, z.B. Lactose, Saccharose, Mannit oder Sorbit, und/oder Cellulosepräparate, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner etwa Carboxymethylstärke, quervernetztes Polyvinylpyrrolidon, Alginsäure oder ein Salz davon, z.B. Natriumalginat.

Zusätzliche Hilfsmittel sind in erster Linie Fliessregulier- und Schmiermittel, z.B. Kieselsäure, Talk, Stearinsäure und/oder Polyethylenglykol, oder Derivate davon.

Sachets sind Behältnisse, z.B. Beutel aus Polyäthylen, kaschiertem Papier oder Aluminium, welche direkt die Granulate, oder auch Pellets, enthalten. Nach dem Öffnen können die Granulate bzw. Pellets entweder unmittelbar eingenommen oder vor der Einnahme mit z.B. Wasser versetzt werden.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Bevorzugt peroral anwendbare pharmazeutische Zusammensetzungen sind z.B. Steckkapseln aus Gelatine sowie weiche, geschlossene Kapseln aus Gelatine und einem Weichmacher, wie z.B. Glycerin oder Sorbit. Die Steckkapseln können das Gemisch Wirkstoff/makrozyklischer Polyether in Form eines Granulats, z.B. im Gemisch mit Füllstoffen, wie Maisstärke, Bindemitteln und/oder Gleitmitteln, wie z.B. Talk, und gegebenenfalls von Stabilisatoren, enthalten. In weichen Kapseln ist das Gemisch Wirkstoff/makrozyklischer Polyether vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Das nachfolgende Beispiel illustriert die vorliegende Erfindung.

Beispiel 1: Gelatinekapseln enthaltend 0,25 g eines Gemisches Wirkstoff/makrozyklischer Polyether können z.B. wie folgt hergestellt werden:

**Zusammensetzung (für 5000 Kapseln)**

| | |
|---|---|
| Dinatriumpamidronat-Pentahydrat (≙ 250 g wasserfreies Dinatrium-pamidronat) | 328,83 g |
| (+)-18-Krone-6-tetracarbonsäure | 1000 g |
| Weizenstärke | 120 g |
| Stearinsäure | 80 g |
| Laktose | 20 g |

Die pulverförmigen Substanzen werden durch ein Sieb mit einer Maschenweite von 0,6 mm getrieben und gemischt. Portionen von je 0,31 g des Gemisches werden mittels einer Kapselfüllmaschine in Gelatine-Kapseln abgefüllt.

## Patentansprüche

1. Eine pharmazeutische Zusammensetzung enthaltend
(1) mindestens ein pharmazeutisch verwendbares Methandiphosphonsäure-Derivat,
(2) mindestens einen makrozyklischen Polyether der Formel II, 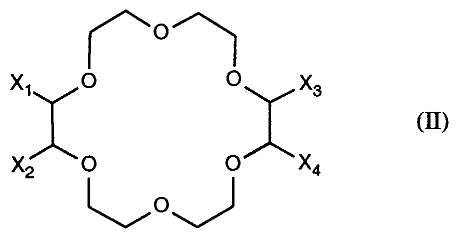 worin X₁-X₄ unabhängig voneinander Carboxy, Carbamoyl oder N-mono- oder N,N-di-substituiertes Carbamoyl bedeuten, oder ein pharmazeutisch annehmbares Salz davon, und
(3) gegebenfalls pharmazeutisch annehmbare Hilfsstoffe.

2. Pharmazeutische Zusammensetzung gemäss Anspruch 1 enthaltend als Methandiphosphonsäure-Derivat mindestens eine Verbindung der Formel I, 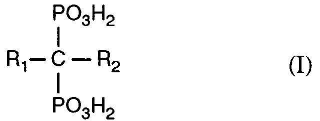 worin
(a) R₁ und R₂ unabhängig voneinander Halogen bedeuten, oder
(b) R₁ Wasserstoff ist und R₂ eine Gruppe Ar-S-, Het₁-NH-, Cyc-NH-, Ar-S-A-N(R')- oder Het₃-S-A-N(R')- bedeutet, worin Ar für unsubstituiertes oder substituiertes Phenyl steht, Het₁ und Het₃ jeweils unsubstituiertes oder substituiertes, monocyclisches 5- oder 6-gliedriges Monoaza-, Diaza- oder Thiaza-aryl bedeuten, welches über ein Ringkohlenstoffatom gebunden ist, Cyc für Cycloakyl steht, A Akylen bedeutet und R' für Wasserstoff oder Niederalkyl steht, oder
(c) R₁ Wasserstoff oder Hydroxy bedeutet und R₂ für -A-R₃ steht, worin A Akylen bedeutet und
R₃ entweder für Het₂ steht, das wie Het₁ definiert ist, aber über ein Ringkohlenstoff- oder ein Ringstickstoffatom gebunden sein kann, oder
R₃ Wasserstoff oder unsubstituiertes oder durch Akyl, Cycloalkyl, Ar-Alkyl, Ar-O-Alkyl, Ar-S-Akyl oder Het₁-Akyl unabhängig voneinander mono- oder disubstituiertes Amino bedeutet, wobei Ar und Het₁ wie oben definiert sind, oder
R₃ für durch unsubstituiertes oder durch Ar substituiertes Akylenamino steht, worin zwei Alkylenkohlenstoffatome zusätzlich über Alkylen miteinander verknüpft sein können und Ar wie oben definiert ist,
oder ein pharmazeutisch annehmbares Salz davon.

3. Pharmazeutische Zusammensetzung gemäss Anspruch 1 enthaltend als Methandiphosphonsäure-Derivat mindestens eine Verbindung der Formel I, worin
(a) R₁ und R₂ unabhängig voneinander Halogen bedeuten, oder
(b) R₁ Wasserstoff ist und R₂ eine Gruppe Ar-S-, Het₁-NH-, Cyc-NH-, Ar-S-A-NH- oder Het₃-S-A-NH- bedeutet, worin Ar für unsubstituiertes oder durch Niederalkyl, Niederalkoxy, Trifluormethyl und/oder Halogen substituiertes Phenyl steht, Het₁ unsubstituiertes oder durch Niederalkyl substituiertes Thiazolyl bedeutet, Cyc für C₃-C₇-Cycloakyl steht, A Niederakylen bedeutet und Het₃ für Thiazolyl oder Pyridyl steht, welches jeweils unsubstituiert oder durch Niederakyl substituiert ist, oder
(c) R₁ Wasserstoff oder Hydroxy bedeutet und R₂ für -A-R₃ steht, worin A Niederakylen bedeutet und
R₃ entweder unsubstituiertes oder durch Niederalkyl substituiertes Imidazolyl, welches über ein Ringkohlenstoff- oder ein Ringstickstoffatom gebunden ist, oder Pyridyl bedeutet, oder
R₃ Wasserstoff oder unsubstituiertes oder durch Niederalkyl, C₃-C₇-Cycloalkyl, Ar-Niederayl, Ar-O-Niederalkyl, Ar-S-Niederakyl oder Pyridylniederalkyl unabhängig voneinander mono- oder di-substituiertes Amino bedeutet, wobei Ar wie oben definiert ist, oder
R₃ für durch unsubstituiertes oder durch Ar substituiertes C₄-C₆-Alkylenamino steht, worin zwei nicht benachbarte Niederalkylenkohlenstoffatome zusätzlich über C₁-C₃-Alkylen miteinander verknüpft sein können und Ar wie oben definiert ist,
oder ein pharmazeutisch annehmbares Salz davon.

4. Pharmazeutische Zusammensetzung gemäss Anspruch 1 enthaltend eine pharmazeutisch verwendbare Methandiphosphonsäure der Formel I, worin
(a) R₁ und R₂ Halogen bedeuten, oder
(b) R₁ Wasserstoff ist und R₂ unsubstituiertes oder durch Halogen substituiertes Phenylthio, unsubstituiertes oder durch Niederalkyl substituiertes Thiazolylamino oder C₅-C₇-Cycloakylamino bedeutet, oder
(c) R₁ Wasserstoff oder Hydroxy bedeutet und R₂ für -A-R₃ steht, worin A C₁-C₇-Alkylen bedeutet und R₃ über ein Ringkohlenstoff- oder Ringstickstoffatom gebundenes, unsubstituiertes oder durch Niederakyl substituiertes Imidazolyl, oder Pyridyl bedeutet, oder
(d) R₁ Hydroxy ist und R₂ für -A-R₃ steht, worin A C₁-C₇-Alkylen bedeutet und R₃ Amino, Di-C₁-C₅-alkylamino, N-C₃-C₇-Cycloakylamino, N-C₁-C₄-Alkyl-N-phenyl-C₁-C₅-akylamino, N-C₁-C₄-Alkyl-N-phenoxy-C₁-C₄-akyl, N-C₁-C₄-Alkyl-N-phenyl-thio-C₁-C₄-alkyl, N-C₁-C₄-Alkyl-N-pyridyl-C₁-C₄-alkylamino; C₄-C₆-Alkylenamino, welches unsubstituiert oder durch Phenyl, das seinerseits unsubstituiert oder durch Halogen substituiert ist, substituiert ist; oder 1,5-Di-C₁-C₄-alkyl-3-aza-bicyclo[3.1.1]-hept-3-yl bedeutet, oder
(e) R₁ Hydroxy ist und R₂ für -A-R₃ steht, worin A C₁-C₄-Alkylen bedeutet und R₃ Wasserstoff ist,
oder ein pharmazeutisch annehmbares Salz davon.

5. Pharmazeutische Zusammensetzung gemäss Anspruch 1 enthaltend eine pharmazeutisch verwendbare Methandiphosphonsäure der Formel I, worin
(a) R₁ und R₂ Chlor bedeuten, oder
(b) R₁ Wasserstoff ist und R₂ unsubstituiertes oder durch Chlor substituiertes Phenylthio, unsubstituiertes oder durch C₁-C₄-Alkyl substituiertes Thiazolylamino oder Cycloheptylamino bedeutet, oder
(c) R₁ Wasserstoff oder Hydroxy bedeutet und R₂ für -A-R₃ steht, worin A Methylen, Ethylen, Propylen oder Pentylen bedeutet und R₃ Imidazol-1-yl, Imidazol-5-yl, 1-Methylimidazol-2-yl, 4-Methyl-imidazol-5-yl, oder 2- oder 3-Pyridyl bedeutet, oder
(d) R₁ Hydroxy ist und R₂ für -A-R₃ steht, worin A Methylen, Ethylen, Propylen oder Pentylen bedeutet und R₃ Amino, Dimethylamino, N-Methyl-N-n-propylamino, N-Methyl-N-n-pentylamino, N-Cycloheptylamino, N-Methyl-N-(2-phenylethyl)-amino, N-Methyl-N-(3-phenylpropyl)-amino oder N-Methyl-N-(5-phenylpentyl)-amino, N-Methyl-N-(3-phenoxypropyl)-amino, N-Methyl-N-(2-phenylthioethyl)-amino, N-Methyl-N-(3-phenylthiopropyl)-amino, N-Methyl-N-[3-(2-pyridyl)-propyl]-amino, Piperidin-1-yl, welches unsubstituiert oder in 4-Stellung durch Phenyl substituiert ist, oder Pyrrolidin-1-yl, welches unsubstituiert oder in 3-Stellung durch 4-Chlorphenyl substituiert ist, oder 1,5-Dimethyl-3-aza-bicyclo[3.1.1]hept-3-yl bedeutet, oder
(e) R₁ Hydroxy ist und R₂ für -A-R₃ steht, worin A Methylen bedeutet und R₃ Wasserstoff ist,
oder ein pharmazeutisch annehmbares Salz davon.

6. Pharmazeutische Zusammensetzung gemäss Anspruch 1 enthaltend eine pharmazeutisch wirksame Methandiphosphonsäure ausgewählt aus der folgenden Gruppe von Verbindungen der Formel I: 4-Amino-1-hydroxy-butan-1,1-diphosphonsäure, 6-Amino-1-hydroxy-hexan- 1,1-diphosphonsäure, 3-(N-Methyl-N-n-pentyl-amino)-1-hydroxy-propan-1,1 -diphosphonsäure, 3-[N-(3-Phenylpropyl)-N-methyl-amino]-1-hydroxy-propan-1,1-diphosphonsäure, 3-(N-Methyl-N-5-phenylpentyl-amino)-1-hydroxypropan-1,1-diphosphonsäure, 3-[N-Methyl-N-3-(2-pyridyl)-propyl-amino]-1-hydroxy-propan-1,1-diphosphonsäure, 1 -Hydroxy-3-[N-methyl-N-(3-phenoxypropyl)-amino]-propan-1,1-diphosphonsäure, 1-Hydroxy-3-[N-methyl-N-(2-phenoxyethyl)-amino]-propan-1,1-diphosphonsäure, 4-(4-Phenyl-piperidin1-yl)-1-hydroxy-butan-1,1-diphosphonsäure, 1-Hydroxy-3-(1 -piperidino)-propan-1,1-diphosphonsäure, 1-Hydroxy-3- [3-(4-chlorphenyl)-pyrrolidin-1-yl]-propan-1, 1-diphosphonsäure, 1-Hydroxy-2-(imidazol-1-yl)-ethan-1,1-diphosphonsäure, 2-(1 -Methyl-imidazol-2-yl)-ethan-1,1-diphosphonsäure, 1-Hydroxy-2-(4-methyl-imidazol-5-yl)-ethan-1,1-diphosphonsäure, 1-Hydroxy-2-(imidazol-5-yl)-ethan-1,1-diphosphonsäure, 1 -Hydroxy-2-(3-pyridyl)-ethan-1,1-diphosphonsäure, 2-(2-Pyridyl)-ethan-1,1-diphosphonsäure, 1-[(5-n-Butyl-2-thiazolyl)-amino]-methan-1,1-diphosphonsäure, 1-[(5-Methyl-2-thiazolyl)-amino]-methan-1,1-diphosphonsäure, 1-[(2-Thiazolyl)-amino]-methan- 1,1-diphosphonsäure, 1-Hydroxy-ethan-1,1-diphosphonsäure, 1-(4-Chlor-phenylthio)-methan-1,1-diphosphonsäure, 1,1-Dichlormethan-1,1-diphosphonsäure, 3-(1,5-Dimethyl-3-azabicyclo[3.1.1]hept-3-yl)-1-hydroxy-propan-1,1-diphosphonsäure, 1-[(5-Ethyl-2-thiazolyl)-amino]-methan-1,1-diphosphonsäure, 3-[N-(2-Phenylethyl)-N-methyl-amino]-1-hydroxy-propan-1,1-diphosphonsäure, 3-[N-(2-Phenylthioethyl)-N-methyl-amino]-1-hydroxy-propan-1,1-diphosphonsäure, 3-[N-(3-Phenylthiopropyl)-N-methyl-amino]-1-hydroxy-propan-1,1-diphosphonsäure, 1-Hydroxy-3-(pyrrolidin-1 -yl)-propan-1,1-diphosphonsäure, 2-(N-Cycloheptylamino)-ethan-1,1-diphosphonsäure, 1-(N-Cycloheptylamino)-methan-1,1-diphosphonsäure, 2-(N,N-Dimethylamino)-1-hydroxy-ethan-1,1-diphosphonsäure, 3-Amino-1-hydroxy-propan-1,1-diphosphonsäure,3-(N,N-Dimethylamino)-1-hydroxypropan-1,1-diphosphonsäure, 3-(N-Methyl-N-n-propyl-amino)-1-hydroxypropan-1,1-diphosphonsäure, 1-[N-(4-Phenylthiobutyl)-amino]-methan-1,1-diphosphonsäure oder 1-{N-[4-(2-Pyridyl)thiobutyl]-amino}-methan- 1,1 -diphosphonsäure, oder jeweils ein pharmazeutisch verwendbares Salz davon.

7. Pharmazeutische Zusammensetzung gemäss Anspruch 1 enthaltend als Methandiphosphonsäure-Derivat eine Verbindung der Formel Ia, 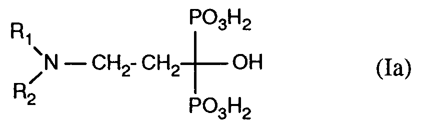 worin R₁ und R₂ unabhängig voneinander Wasserstoff oder C₁-C₃-Alkyl bedeuten, oder ein pharmazeutisch verwendbares Salz davon.

8. Pharmazeutische Zusammensetzung gemäss Anspruch 1 enthaltend als Methandiphosphonsäure-Derivat 3-Amino-1-hydroxy-propan-1,1 -diphosphonsäure oder ein pharmazeutisch anwendbares Salz davon.

9. Pharmazeutische Zusammensetzung gemäss Anspruch 8 enthaltend als Methandiphosphonsäure-Derivat das Dinatriumsalz der 3-Amino-1 -hydroxy-propan-1,1-diphosphonsäure.

10. Pharmazeutische Zusammensetzung gemäss Anspruch 1 enthaltend als Methandiphosphonsäure-Derivat 3-(N,N-Dimethylamino)-1-hydroxy-propan-1,1 -diphosphonsäure oder ein pharmazeutisch anwendbares Salz davon.

11. Pharmazeutische Zusammensetzung gemäss Anspruch 1 enthaltend als Methandiphosphonsäure-Derivat 3-(N-Methyl-N-n-propyl-amino)-1-hydroxy-propan-1,1-diphosphonsäure oder ein pharmazeutisch anwendbares Salz davon.

12. Pharmazeutische Zusammensetzung gemäss Anspruch 1 enthaltend als Methandiphosphonsäure-Derivat die 1-Hydroxy-2-(imidazol-1-yl)-ethan-1,1-diphosphonsäure oder ein pharmazeutisch anwendbares Salz davon.

13. Pharmazeutische Zusammensetzung gemäss Anspruch 1 enthaltend als Methandiphosphonsäure-Derivat die 3-[N-(2-Phenylthioethyl)-N-methyl-amino]-1-hydroxy-propan-1,1-diphosphonsäure oder ein pharmazeutisch anwendbares Salz davon.

14. Pharmazeutische Zusammensetzung gemäss Anspruch 1 enthaltend als Methandiphosphonsäure-Derivat die 3-[N-(3-Phenylthiopropyl)-N-methyl-amino]- 1-hydroxy-propan-1,1-diphosphonsäure oder ein pharmazeutisch anwendbares Salz davon.

15. Pharmazeutische Zusammensetzung gemäss einem der Ansprüche 1-14, enthaltend als makrozyklischen Polyether der Formel II eine Verbindung der Formel IIa, 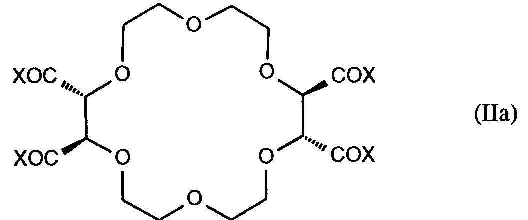 worin die Reste X identisch sind und für Hydroxy stehen, sowie pharmazeutisch verwendbare Salze davon.

16. Pharmazeutische Zusammensetzung gemäss Anspruch 9 enthaltend das Dinatriumsalz der 3-Amino-1-hydroxy-propan-1,1-diphosphonsäure und die (+)-1,4,7,10,13,16-Hexaoxa-cyclooctadecan-2,3,11,12-tetracarbonsäure, oder ein pharmazeutisch anwendbares Salz davon.

17. Pharmazeutische Zusammensetzung gemäss einem der Ansprüche 1-16, dadurch gekennzeichnet, dass das (die) pharmazeutisch verwendbare(n) Methandiphosphonsäure-Derivat(e) und der (die) makrozyklische(n) Polyether in einem Verhältnis von 1:1 bis 1:16 vorliegen.

18. Pharmazeutische Zusammensetzung gemäss einem der Ansprüche 1-16, dadurch gekennzeichnet, dass das (die) pharmazeutisch verwendbare(n) Methandiphosphonsäure-Derivat(e) und der (die) makrozyklische(n) Polyether in einem Verhältnis von 1:1 bis 1:8 vorliegen.

19. Pharmazeutische Zusammensetzung gemäss einem der Ansprüche 1-16, dadurch gekennzeichnet, dass das (die) pharmazeutisch verwendbare(n) Methandiphosphonsäure-Derivat(e) und der (die) makrozyklische(n) Polyether in einem Verhältnis von 1:2 bis 1:6 vorliegen.

20. Verfahren zur Steigerung der peroralen Absorption von pharmazeutisch verwendbaren Methandiphosphonsäure-Derivaten, welches dadurch gekennzeichnet ist, dass man ein pharmazeutisch verwendbares Methandiphosphonsäure-Derivat zusammen mit einem makrozyklischen Polyether der Formel II wie in Anspruch 1 definiert, oder einem pharmazeutisch annehmbares Salz davon, in eine pharmazeutische Zusammensetzung einarbeitet.

21. Verwendung von pharmazeutischen Zusammensetzungen gemäss einem der Ansprüche 1-19 zur Behandlung von Hypercalcämie und osteolytischen Knochenmetastasen.

22. Pharmazeutische Zusammensetzung gemäss einem der Ansprüche 1-19 zur Anwendung in einem therapeutischen Verfahren des menschlichen oder tierischen Körpers.

23. Pharmazeutische Zusammensetzung gemäss einem der Ansprüche 1-19 zur Anwendung in einem therapeutischen Verfahren bei der Behebung von Störungen des Calcium- und/oder Phosphatstoffwechsels.
